# FASCICULE DE BREVET EUROPEEN

(11) **EP 0 812 345 B1**
(45) Date de publication et mention de la délivrance du brevet: **26.09.2001**
(21) Numéro de dépôt: 96944086.6
(22) Date de dépôt: 27.12.1996
(51) Int. Cl.: C10G 45/32

(54) **PROCEDE D'HYDROGENATION SELECTIVE D'UNE COUPE D'HYDROCARBURES**
METHODE ZUR SELEKTIVEN HYDRIERUNG EINER DESTILLATIONSFRAKTION VON KOHLENWASSERSTOFFEN
METHOD FOR SELECTIVE HYDROGENATION OF A DISTILLATION CUT OF HYDROCARBONS

(30) Priorité: 27.12.1995 FR 9515530
(43) Date de publication de la demande: 17.12.1997
(73) Titulaire: INSTITUT FRANCAIS DU PETROLE, 92852 Rueil-Malmaison Cedex (FR)
(72) Inventeur: COSYNS, Jean, F-78580 Maule (FR); DIDILLON, Blaise, F-92500 Rueil-Malmaison (FR); NOCCA, Jean-Luc, Houston, TX 77042 (US); LEBAS, Etienne, F-92500 Rueil-Malmaison (FR); MONTECOT, Françoise, F-78340 Les Clayes-sous-Bois (FR)
(86) Numéro de dépôt international: FR9602085
(87) Numéro de publication internationale: WO9724413

(56) Documents cités:
- EP-A- 0 554 151
- EP-A- 0 556 025
- WO-A-96/06900
- US-A- 4 847 430
- US-A- 5 368 691
- US-A- 5 461 178

## Description

L'invention concerne un procédé de traitement d'une charge constituée en partie par des hydrocarbures contenant au moins 3 atomes de carbone par molécule, parmi lesquels des hydrocarbures acétyléniques et dioléfiniques, ainsi que des traces d'arsine (AsH₃), de l'oxysulfure de carbone (COS) et des alkylthiols (mercaptans). Ce procédé consiste à traiter ladite charge dans une colonne de distillation, comportant une zone d'épuisement et une zone de rectification associée à une zone réactionnelle d'hydrogénation comportant au moins un lit catalytique dans laquelle on réalise l'hydrogénation d'au moins une partie des composés acétyléniques et dioléfinique contenus, dans la charge, la charge de ladite zone réactionnelle étant prélevée à la hauteur d'un niveau de prélèvement et représentant au moins une partie du liquide coulant dans la zone de rectification, l'effluent de la zone réactionnelle étant au moins en partie réintroduit dans la zone de rectification de manière à assurer la continuité de la distillation et de façon à sortir en tête de la colonne de distillation un effluent très appauvri en hydrocarbures acétyléniques et dioléfiniques comprenant au plus 5 atomes de carbone et en fond de colonne de distillation un effluent également appauvri en ces composés.

Le procédé de l'invention permet aussi d'effectuer dans la zone réactionnelle une réaction d'addition des alkylthiols légers (méthyl-mercaptan et éthyl-mercaptan) sur les hydrocarbures dioléfiniques comprennent au plus 5 atomes de carbone, produisant des thioéthers lourds comprenant au moins 5 atomes de carbone et bouillant dans la gamme des hydrocarbures comprenant au moins 6 atomes de carbone. L'effluent de la zone réactionnelle est réintroduit dans la zone de rectification de façon à sortir en tête de la zone de rectification, un effluent très appauvri en alkylthiols légers et en fond de colonne de distillation un effluent contenant les thioéthers comprenant au moins 5 atomes de carbone.

Les divers procédés de craquage utilisés dans l'industrie pétrolière tels que le craquage thermique, la cokéfaction, la viscoréduction, le craquage catalytique et le vapocraquage produisent des grandes quantités de coupes d'hydrocarbures légers oléfiniques contenant en outre des hydrocarbures acétyléniques et dioléfiniques.

Les utilisations ultérieures de ces oléfines pour la fabrication de polymères ou de carburant par exemple, nécessitent l'élimination sélective aussi complète que possible des hydrocarbures acétyléniques et dioléfiniques.

La technique habituelle pour cette purification est l'hydrogénation sélective réalisée sous courant d'hydrogène dans un réacteur comportant un lit fixe de catalyseur constitué par un métal du groupe VIII. La charge qui arrive à température ambiante est généralement pompée et réchauffée et réintroduite dans le réacteur d'hydrogénation. L'hydrogène nécessaire est ajouté de toute manière appropriée. A la sortie du réacteur, l'excès d'hydrogène ainsi que les hydrocarbures gazeux contenus dans le gaz d'alimentation sont enlevés de la coupe liquide hydrocarbonée de toute manière appropriée, mais généralement dans un ballon séparateur suivi si nécessaire d'une colonne d'étêtage.

Il a été décrit récemment, notamment dans la demande de brevet européen EP 0 556 025 A1, un procédé d'hydrogénation sélective des dioléfines dans une coupe d'hydrocarbures oléfiniques légers de raffinerie. Dans cette technologie, l'hydrogénation des dioléfines est interne à la zone de rectification de la colonne de distillation, le catalyseur lui-même agissant comme structure de distillation. Cette manière de faire permet une économie d'appareillage. Mais selon ce type de technologie où la réaction et la distillation procèdent simultanément dans le même espace physique, la phase liquide descend à travers tout le lit catalytique de la zone réactionnelle en écoulement ruisselant en filets de liquide. La phase gazeuse contenant la fraction de charge vaporisée et l'hydrogène monte au travers dudit lit catalytique dans des colonnes de gaz. L'hydrogène nécessaire à la réaction se trouvant dans le phase gazeuse, l'hydrogénation se déroulera essentiellement dans cette phase sur la partie du catalyseur en contact avec la fraction des hydrocarbures se trouvant dans la phase gazeuse.

Dans cette technologie, ce sont donc les hydrocarbures les plus légers qui seront préférentiellement hydrogénés. Ainsi, dans la demande de brevet citée, ce sont les hydrocarbures à cinq atomes de carbone contenus dans l'essence, qui comprend également des hydrocarbures à au moins 6 atomes de carbone. La réaction d'hydrogénation est en fait réalisée dans la zone de rectification du dépentaniseur. Ce sont donc les dioléfines présentes dans la coupe C₅, passant en phase gazeuse, qui seront préférentiellement hydrogénées.

Une telle technologie n'est pas du tout généralisable et présente dans certains cas des inconvénients tels qu'elle en devient impraticable. Ainsi par exemple dans le procédé de craquage catalytique destiné à produire de l'essence à partir de fractions lourdes pétrolières, on ne peut éviter de produire également une quantité importante d'hydrocarbures gazeux essentiellement oléfiniques, mais contenant également des faibles pourcentages de dioléfines.

Dans le mode le plus général de séparation des produits du craquage catalytique, on réalise d'abord un fractionnement, appelé "primaire", des produits de la section réactionnelle du craquage catalytique en gaz légers, en gaz liquéfiables mélangés à l'essence et en produits plus lourds que l'essence. La fraction de coeur qui contient en général les hydrocarbures à au moins trois atomes de carbone jusqu'aux hydrocarbures à dix atomes de carbone est ensuite envoyée dans un débutaniseur qui sépare en fond l'essence et en tête le mélange des hydrocarbures à trois et quatre atomes de carbone généralement très riche en propylène et butènes et contenant des petites quantités de dioléfines, principalement du butadiène. Le mélange contient également de nombreuses impuretés, telles que principalement l'oxysulfure de carbone (COS) et le méthylthiof (CH₃SH) mais aussi très souvent des traces d'arsine (AsH₃). De telles impuretés désactivent fortement le catalyseur d'hydrogénation lorsque celui-ci opère sur le mélange précité et il est nécessaire de procéder au préalable à un traitement de purification qui élimine l'arsine et le COS. En conséquence, le procédé décrit dans la demande de brevet EP 0 556 025 A1 qui consiste à placer le catalyseur dans la zone de rectification qui voit passer simultanément la phase liquide descendante et la phase gazeuse ascendante ne peut que se révéler impraticable, car le catalyseur sera empoisonné par AsH₃ et COS présents principalement en phase gazeuse, leurs points d'ébullition étant respectivement de -55 et -50°C.

Le procédé proposé dans l'invention permet d'éviter les graves inconvénients précités.

Il consiste à traiter une charge, provenant par exemple d'un procédé de craquage et contenant des hydrocarbures comportant de 3 à 10 atomes de carbone et comprenant en proportions généralement faibles des hydrocarbures acétyléniques et dioléfiniques ainsi que des traces d'arsine (AsH3) de l'oxysulfure de carbone (COS) et des alkylthiols (mercaptans) par passage dans une zone de distillation comportant une zone d'épuisement et une zone de rectification, ladite zone de distillation étant en communication avec au moins une zone réactionnelle d'hydrogénation sélective constituée par au moins un lit catalytique, dans laquelle on réalise l'hydrogénation d'au moins une partie des hydrocarbures acétyléniques et dioléfiniques contenus dans ladite charge en présence d'un flux gazeux riche en hydrogène, ledit procédé étant caractérisé en ce que la charge de la (ou des) zone(s) réactionnelle(s) est prélevée à hauteur d'un plateau de prélèvement et représente la majeure partie du liquide coulant dans la zone de rectification et de façon préférée coulant à un niveau intermédiaire de la zone de rectification, l'effluent de la zone réactionnelle étant au moins en majeure partie réintroduit dans la zone de rectification de manière à assurer la continuité de la distillation, en ce qu'au moins une zone réactionnelle d'hydrogénation sélective est interne à la zone de distillation et en ce que la vapeur de distillation est séparée du liquide à hydrogéner pour toute zone réactionnelle interne ou externe à ladite zone de distillation.

Le procédé est conduit de préférence de façon à sortir finalement en tête de la colonne de distillation un effluent très appauvri en hydrocarbures acétyléniques comprenant au plus 5 atomes de carbone et en fond de colonne de distillation un effluent également appauvri en ces composés.

La colonne de distillation comprend généralement un interne de rectification choisi dans le groupe formé par les plateaux, les garnissages en vrac et les garnissages structurés, ainsi qu'il est connue de l'homme de métier, tel que l'efficacité globale totale est généralement au moins égale à cinq étapes théoriques.

La zone réactionnelle d'hydrogénation comprend généralement au moins un lit catalytique d'hydrogénation, de préférence de 1 à 4 lits catalytiques ; dans le cas où elle comporte au moins deux lits catalytiques, ces lits peuvent être séparés par au moins un interne de rectification. La zone réactionnelle d'hydrogénation réalise au moins partiellement l'hydrogénation des composés acétyléniques et dioléfiniques comportant de 3 à 5 atomes de carbone présents dans la charge de telle façon que la teneur en ces composés soit inférieure à une certaine valeur déterminée à l'avance.

Dans le procédé de l'invention, la zone réactionnelle d'hydrogénation est au moins en partie, de préférence en totalité, interne à la colonne de distillation. Alors, pour la partie de la zone réactionnelle interne à la colonne de distillation, le prélèvement de liquide est fait naturellement par écoulement dans la partie de la zone réactionnelle interne à la colonne de distillation, et la réintroduction de liquide en colonne de distillation se fait aussi naturellement par écoulement du liquide à partir de la zone réactionnelle interne à la colonne de distillation. De plus, le procédé selon l'invention est mis en oeuvre de préférence de façon que l'écoulement du liquide à hydrogéner soit co-courant à l'écoulement du flux gazeux comprenant de l'hydrogène, pour tout lit catalytique de la partie interne de la zone d'hydrogénation et que la vapeur de distillation soit séparée dudit liquide, pour tout lit catalytique de la partie interne de la zone d'hydrogénation.

De façon particulière, le procédé de l'invention peut être tel que la zone d'hydrogénation est partiellement incorporée dans la colonne de distillation, c'est-à-dire interne à la colonne de distillation, et partiellement externe à la colonne de distillation. Selon un tel mode de réalisation, la zone d'hydrogénation comprend au moins deux lits catalytiques, au moins un lit catalytique étant interne à la colonne de distillation, et au moins un autre lit catalytique étant externe à la colonne de distillation. Dans le cas où la partie externe de la zone d'hydrogénation comporte au moins deux lits catalytiques, chaque lit catalytique est alimenté par un seul niveau de prélèvement, de préférence associé à un seul niveau où l'effluent dudit lit catalytique de la partie externe de la zone d'hydrogénation est réintroduit, ledit niveau de prélèvement étant distinct du niveau de prélèvement qui alimente l'(es) autre(s) lit(s) catalytique(s). Généralement, le liquide à hydrogéner, soit partiellement, soit totalement, circule d'abord dans la partie externe de la zone d'hydrogénation, puis dans la partie interne de ladite zone. La partie de la zone réactionnelle interne à la colonne de distillation a les caractéristiques décrites dans le premier mode de réalisation, la partie de la zone réactionnelle externe à la colonne de distillation a les caractéristiques décrites dans le deuxième mode de réalisation.

Selon un autre mode de réalisation de l'invention, indépendamment ou non des modes de réalisation précédents, le procédé selon l'invention est tel que l'écoulement du liquide à hydrogéner est co-courant ou contre-courant, de préférence co-courant, à l'écoulement du flux gazeux comprenant de l'hydrogène, pour tout lit catalytique de la zone d'hydrogénation.

Pour la réalisation de l'hydrogénation selon le procédé de l'invention, le rapport molaire théorique d'hydrogène nécessaire pour la conversion désirée des hydrocarbures acétyléniques et dioléfiniques est égal à 1. La quantité d'hydrogène distribué dans le flux gazeux, avant ou dans la zone d'hydrogénation est généralement en léger excès par rapport à cette stoechiométrie pour tenir compte du fait que l'hydrogénation n'est pas parfaitement sélective et qu'une petite partie des oléfines présentes est également hydrogénée. De plus, un peu d'hydrogène peut traverser la zone catalytique sans être consommé. Cet hydrogène en excès, si il en existe, peut être avantageusement récupéré, soit par compression et réutilisation dans la zone d'hydrogénation, soit envoyé vers une autre installation consommatrice d'hydrogène.

L'hydrogène utilisé dans le procédé selon l'invention peut provenir de toutes sources produisant de l'hydrogène à au moins 50 % volume de pureté, de préférence au moins 80 % volume de pureté et de façon encore plus préférée au moins 90 % volume de pureté. Par exemple, on peut citer l'hydrogène provenant des procédés de reformage catalytique, de méthanation, de P.S.A. (adsorption par alternance de pression), de génération électrochimique, de vapocraquage ou de reformage à la vapeur.

Généralement, et de façon préférée, les conditions opératoires sont judicieusement choisies en relation avec la nature de la charge et avec d'autres paramètres connus du spécialiste de la distillation tel que le rapport distillat/charge, de telle manière que l'effluent de tête de la colonne de distillation soit pratiquement exempt d'hydrocarbures acétyléniques et dioléfiniques comprenant au plus 5 atomes de carbone.

Suivant la technologie de l'invention, le catalyseur est disposé de telle façon que la réaction et la distillation procèdent de manière indépendante et consécutive, comme l'enseignent par exemple les brevets US-A-4.847.430, US-A-5.130.102 et US-A-5.368.691, la vapeur de la colonne de distillation ne traversant pratiquement pas tout lit catalytique de la zone réactionnelle. Ainsi, le procédé selon l'invention est généralement tel que l'écoulement du liquide à hydrogéner est co-courant à l'écoulement du flux gazeux comprenant de l'hydrogène et tel que la vapeur de distillation n'est pratiquement pas en contact avec le catalyseur (ce qui se traduit généralement en pratique par le fait que ladite vapeur est séparée dudit liquide à hydrogéner), pour tout lit catalytique de la partie interne de la zone d'hydrogénation. Dans la technologie de l'invention, tout lit catalytique de la partie de la zone réactionnelle qui est dans la colonne de distillation est généralement tel que le flux gazeux comprenant de l'hydrogène et le flux du liquide qui va réagir circulent à co-courant, généralement ascendant, à travers ledit lit, même si globalement, dans la colonne de distillation catalytique, le flux gazeux comprenant de l'hydrogène et le flux du liquide qui va réagir circulent à contre-courant. De tels systèmes comportent généralement au moins un dispositif de distribution de liquide, qui peut être par exemple un répartiteur de liquide, dans tout lit catalytique de la zone réactionnelle. Néanmoins, dans la mesure où ces technologies ont été conçues pour des réactions catalytiques intervenant entre des réactifs liquides, elles ne peuvent convenir sans modification pour une réaction catalytique d'hydrogénation, pour laquelle l'un des réactifs, l'hydrogène, est à l'état gazeux. Pour tout lit catalytique de la partie interne de la zone d'hydrogénation, il est donc généralement nécessaire d'adjoindre un dispositif de distribution du flux gazeux comprenant de l'hydrogène, par exemple selon l'une des trois techniques décrites ci-après. Ainsi, la partie interne de la zone d'hydrogénation comporte au moins un dispositif de distribution de liquide et au moins un dispositif de distribution du flux gazeux comprenant de l'hydrogène, pour tout lit catalytique de la partie interne de la zone d'hydrogénation. Selon une première technique, le dispositif de distribution du flux gazeux comprenant de l'hydrogène est disposé avant le dispositif de distribution de liquide, et donc avant le lit catalytique. Selon une deuxième technique, le dispositif de distribution du flux gazeux comprenant de l'hydrogène est disposé au niveau du dispositif de distribution de liquide, de telle façon que le flux gazeux comprenant de l'hydrogène soit introduit dans le liquide avant le lit catalytique. Selon une troisième technique, le dispositif de distribution de flux gazeux comprenant de l'hydrogène est disposé après le dispositif de distribution de liquide, et donc au sein du lit catalytique, de préférence non loin dudit dispositif de distribution du liquide dans ledit lit catalytique. Les termes "avant" et "après" utilisés ci-dessus s'entendent par rapport au sens de circulation du liquide qui va traverser le lit catalytique, c'est-à-dire généralement dans le sens ascendant.

Une des réalisations préférées du procédé selon l'invention est telle que le catalyseur de la partie interne de la zone d'hydrogénation est disposé dans la zone réactionnelle suivant le dispositif de base décrit dans le brevet US-A-5.368.691, aménagé de manière que tout lit catalytique interne à la colonne de distillation soit alimenté par un flux gazeux comprenant de l'hydrogène, régulièrement distribué à sa base, par exemple selon l'une des trois techniques décrites ci-dessus. Suivant cette technologie, si la zone d'hydrogénation est en totalité interne à ladite colonne, le catalyseur compris dans tout lit catalytique, interne à la colonne de distillation, est alors en contact avec une phase liquide ascendante, générée par le reflux introduit au sommet de la colonne de distillation, et avec le flux gazeux comprenant de l'hydrogène qui circule dans le même sens que le liquide ; le contact avec la phase vapeur de la distillation est évité en faisan transiter cette dernière par au moins une cheminée spécialement aménagée.

Lorsque la zone d'hydrogénation est au moins en partie interne à la colonne de distillation, les conditions opératoires de la partie de la zone d'hydrogénation interne à la colonne de distillation sont liées aux conditions opératoires de la distillation. La distillation est conduite de manière que son produit de fond contienne de préférence la majeure partie des hydrocarbures à au moins 5 atomes de carbone. Elle est réalisée sous une pression généralement comprise entre 2 et 25 bar, de préférence entre 4 et 15 bar (1 bar = 10⁵ Pa), avec un taux de reflux compris entre 0,5 et 10, et de préférence compris entre 3 et 6. La température de tête de zone est comprise généralement entre 20 et 100°C la température de fond de zone est comprise généralement entre 100 et 300°C. La réaction d'hydrogénation est conduite dans des conditions qui sont le plus généralement intermédiaires entre celles établies en tête et en fond de colonne de distillation, à une température comprise entre 50 et 150°C, et de préférence comprise entre 60 et 100°C, et à une pression comprise entre 2 et 20 bar, de préférence entre 5 et 15 bar. Le liquide soumis à l'hydrogénation est alimenté par un flux gazeux comprenant de l'hydrogène dont le débit dépend de la concentration en hydrocarbures acétyléniques et dioléfiniques dans ledit liquide comportant au plus 5 atomes de carbones par molécule de la charge de la colonne de distillation II est généralement au moins égal au débit correspondant à la stoechiométrie des réactions d'hydrogénation en jeu et au plus égal au débit correspondant à 5 fois la stoechiométrie, de préférence compris entre 1 et à 3 fois la stoechiométrie.

Lorsque la zone d'hydrogénation est en partie externe à la colonne de distillation, le catalyseur disposé dans ladite partie externe l'est suivant toute technologie connue de l'homme du métier dans des conditions opératoires (température, pression,...) indépendantes ou non, de préférence indépendantes, des conditions opératoires de la colonne de distillation.

Dans la partie de la zone d'hydrogénation externe à la colonne de distillation, les conditions opératoires sont généralement les suivantes : la pression requise pour cette étape d'hydrogénation est généralement comprise entre 1 et 60 bar, de préférence entre 2 et 25 bar. La température opératoire de la partie externe de la zone d'hydrogénation est généralement comprise entre 50 et 150°C, de préférence entre 60 et 120°C. La vitesse spatiale au sein de la partie externe de ladite zone d'hydrogénation, calculée par rapport au catalyseur, est généralement comprise entre 1 et 50 et plus particulièrement entre 1 et 30 h⁻¹ (volume de charge par volume de catalyseur et par heure). Le débit d'hydrogène correspondant à la stoechiométrie des réactions d'hydrogène en jeu est compris entre 0,5 et 10 fois ladite stoechiométrie, de préférence entre 1 et 3 fois ladite stoechiométrie. Mais les conditions de température et de pression peuvent aussi, dans le cadre du procédé de la présente invention, être comprise entre celles qui sont établies en tête et en fond de colonne de distillation.

De façon plus générale, quelle que soit la position de la zone d'hydrogénation selon le procédé de la présente invention comprend généralement au moins un métal choisi dans le groupe VIII déposé sur un support.
Le catalyseur est de préférence un catalyseur au palladium.

On utilisera notamment les catalyseurs vendus par la société PROCATALYSE sous des références LD 265® et LD 277-3® .

L'exemple qui suit illustre l'invention.

### Exemple

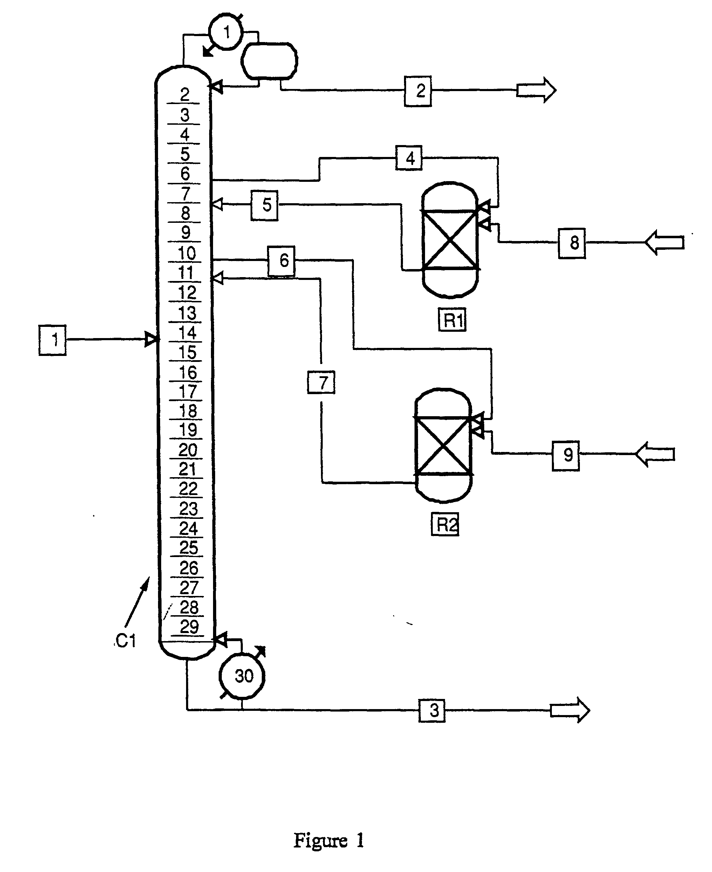

L'exemple est conduit selon le schéma de procédé représenté sur la figure 1.

Une coupe issue d'une unité de craquage catalytique est introduite dans une zone de distillation et de réaction constituée d'une colonne C1, comprenant 30 plateaux théoriques (y compris le condenseur 1 et le rebouilleur 30). L'alimentation se fait au niveau du plateau 14 par la ligne 1.

L'unité de distillation réactive comporte deux zones d'hydrogénation R1 et R2 externes à la colonne.

Les zones d'hydrogénation R1 et R2 sont alimentées par des soutirages effectués respectivement au niveau des plateaux 6 et 10, par les lignes 4 et 6 ; l'effluent de chacun des réacteurs extérieurs est réintroduit sur le plateau inférieur au plateau de soutirage alimentant le réacteur extérieur considéré, c'est à dire respectivement au niveau des plateaux 7 et 11, par les lignes 5 et 7.

Les deux réacteurs contiennent chacun un catalyseur au palladium vendu par la société PROCATALYSE sous la dénomination LD-265®. Ils reçoivent chacun un flux d'hydrogène, respectivement par les lignes 8 et 9.

L'effluent de tête de la colonne C1 est évacué, après condensation, par la ligne 2 et l'effluent de fond par la ligne 3.

Les conditions opératoires sont les suivantes :
- débit d'alimentation de la colonne C1 : 2146,84 kmol/h,
- pression en tête de colonne : 11,3 bar absolu,
- pression en fond de colonne : 11,5 bar absolu,
- température de l'alimentation de la colonne : 111°C,
- température en tête de colonne : 44°C,
- température en fond de colonne : 170°C,
- température du réacteur R1 : 73°C,
- débit dans le réacteur R1 : 1200 kmole/h,
- température du réacteur R2 : 87°C,
- débit dans le réacteur R2 : 1000 kmole/h.

Avec cette configuration et dans ces conditions opératoires, le procédé a conduit aux résultats suivants:

**Tableau 1**

| | Charge (% mol) | Tête (% mol) | Fond (% mol) |
|---|---|---|---|
| H₂O | 0,0002 | 0,0006 | 0 |
| N₂ | 0 | 0 | 0 |
| CO | 0 | 0 | 0 |
| CO₂ | 0 | 0 | 0 |
| H₂S | 0,0433 | 0,1233 | 0 |
| H₂ | 0 | 0 | 0 |
| C1 | 0 | 0 | 0 |
| C2 | 0,0251 | 0,0715 | 0 |
| Ethylène | 0,0003 | 0,0007 | 0 |
| C3 | 16,9195 | 48,1552 | 0,0001 |
| i C4 | 3,9905 | 11,2647 | 0,0488 |
| n C4 | 1,6451 | 4,1906 | 0,2326 |
| Butène-1 | 12,8531 | 35,6012 | 0,5067 |
| iC5 | 5,6754 | 0,3211 | 8,491 |
| n C5 | 1,1646 | 0,0178 | 1,7941 |
| Cyclopentane | 0,3013 | 0,0001 | 0,4668 |
| Pentène-1 | 12,6208 | 0,1716 | 19,5755 |
| 50-70 | 10,6238 | 0 | 16,4159 |
| 70-90 | 9,1166 | 0 | 14,0859 |
| 90-110 | 7,7906 | 0 | 12,0372 |
| 110-130 | 6,6165 | 0 | 10,223 |
| 130-140 | 2,9066 | 0 | 4,4909 |
| 140-150 | 2,6237 | 0 | 4,0538 |
| 150-160 | 2,239 | 0 | 3,4594 |
| 160-170 | 1,6277 | 0 | 2,5150 |
| 170-180 | 0,7715 | 0 | 1,1921 |
| 180-190 | 0,2092 | 0 | 0,3232 |
| 190-200 | 0,0425 | 0 | 0,0656 |
| 200-210 | 0,0076 | 0 | 0,0118 |
| 210-220 | 0,0012 | 0 | 0,0018 |
| 220-230 | 0,0001 | 0 | 0,0002 |
| 230-250 | 0 | 0 | 0 |
| 250-270 | 0 | 0 | 0 |
| 270-290 | 0 | 0 | 0 |
| 1,2-butadiène | 0 | 0 | 0 |
| 1,3-butadiène | 0,1845 | 0,0816 | 0,0087 |
| Débit (kmol/h) | 2146,84 | 754,156 | 1389,46 |

On a obtenu une conversion du butadiène de 80%. Par ailleurs, la teneur en butadiène-1,3 dans le produit de tête de colonne est de 800 ppm en mole.

## Revendications

1. Procédé de traitement d'une charge comprenant des hydrocarbures de 3 à 10 atomes de carbone par molécule, parmi lesquels des hydrocarbures acétyléniques et dioléfiniques par passage dans une zone de distillation comportant une zone d'épuisement et une zone de rectification, qui produit une vapeur et un liquide, ladite zone de distillation étant en communication avec au moins une zone réactionnelle d'hydrogénation sélective constituée par au moins un lit catalytique, dans laquelle on réalise l'hydrogénation d'au moins une partie des hydrocarbures acétyléniques et dioléfiniques contenues dans ladite charge en présence d'un flux gazeux riche en hydrogène, le dit procédé étant **caractérisé en ce que** la charge de la (ou des) zone(s) réactionnelle(s) est prélevée à hauteur d'un plateau de prélèvement et représente au moins la majeure partie du liquide coulant dans la zone de rectification, l'effluent de la zone réactionnelle étant au moins en majeure partie réintroduit dans la zone de rectification de manière à assurer la continuité de la distillation, **en ce que** au moins une zone réactionnelle d'hydrogénation sélective est interne à la zone de distillation et **en ce que** la vapeur de distillation est séparée du liquide à hydrogéner pour toute zone réactionnelle interne ou externe à ladite zone de distillation.

2. Procédé selon la revendication 1, **caractérisé en ce que** la charge de la zone réactionnelle représente la majeure partie du liquide coulant à un niveau intermédiaire de la zone de rectification.

3. Procédé selon la revendication 1 ou 2, **caractérisé en ce qu'**il est effectué de façon à sortir finalement en tête de la colonne de distillation un effluent très appauvri en hydrocarbures acétyléniques et / ou dioléfiniques comprenant au plus 5 atomes de carbone et en fond de colonne de distillation un effluent également appauvri en ces composés.

4. Procédé selon l'une des revendications 1 à 3, **caractérisé en ce que** chaque zone d'hydrogénation sélective comprend de 1 à 4 lits catalytiques.

5. Procédé selon l'une des revendications 1 à 4, **caractérisé en ce que** la zone d'hydrogénation sélective associée à la zone de distillation est totalement interne à ladite zone de distillation.

6. Procédé selon l'une des revendications 1 à 4, **caractérisé en ce qu'**il comprend au moins une zone d'hydrogénation sélective interne et au moins une zone d'hydrogénation externe à ladite zone de distillation.

7. Procédé selon l'une des revendications 1 à 6, **caractérisé en ce que** le catalyseur est un catalyseur supporté comprenant au moins un métal noble du groupe VIII.

8. Procédé selon la revendication 7, **caractérisé en ce que** le catalyseur est un catalyseur au palladium.

## Patentansprüche

1. Verfahren zur Behandlung einer Charge, die 3 bis 10 Kohlenstoffatome pro Molekül umfasst, unter ihnen acetylenische und diolefinische Kohlenwasserstoffe, durch Durchleiten in einer Destillationszone, die eine Erschöpfungszone und eine Rektifikationszone umfasst und die einen Dampf und eine Flüssigkeit erzeugt, wobei diese Destillationszone in Verbindung mit wenigstens einer Reaktionszone der selektiven Hydrierung steht, die durch wenigstens ein katalytisches Bett gebildet ist, in dem man die Hydrierung wenigstens eines Teils der acetylenischen und diolefinischen Kohlenwasserstoffe, die in der Charge enthalten sind, in Gegenwart eines gasförmigen wasserstoffreichen Flusses verwirklicht, wobei dieses Verfahren **dadurch gekennzeichnet ist, dass** die Charge der Reaktionszone(n) in Höhe eines Entnahmebodens entnommen wird und wenigstens den größeren Teil der in der Rektifikationszone fließenden Flüssigkeit darstellt, wobei der Abstrom der Reaktionszone wenigstens zum größeren Teil wieder in die Rektifikationszone eingeführt wird, um die Kontinuität der Destillation sicherzustellen, **dadurch**, **dass** wenigstens eine Reaktionszone selektiver Hydrierung innerhalb der Destillationszone liegt und dadurch, **dass** der Destillationsdampf von der zu hydrierenden Flüssigkeit für jede Reaktionszone innerhalb oder außerhalb der Destillationszone getrennt wird.

2. Verfahren nach Anspruch 1, **dadurch gekennzeichnet, dass** die Charge der Reaktionszone den größeren Teil der auf einem Zwischenniveau der Rektifikationszone fließenden Flüssigkeit darstellt.

3. Verfahren nach einem der Ansprüche 1 oder 2, **dadurch gekennzeichnet, dass** es in einer Weise ausgeführt wird, **dass** letztlich am Kopf der Destillationskolonne ein an acetylenischen und/oder diolefinischen Kohlenwasserstoffen, die höchstens 5 Kohlenstoffatome umfassen, sehr verarmter Abstrom austritt und am Boden der Destillationskolonne ein ebenfalls an diesen Verbindungen verarmter Abstrom.

4. Verfahren nach einem der Ansprüche 1 bis 3, **dadurch gekennzeichnet, dass** jede Zone selektiver Hydrierung 1 bis 4 katalytische Betten umfasst.

5. Verfahren nach einem der Ansprüche 1 bis 4, **dadurch gekennzeichnet, dass** die Zone selektiver Hydrierung, die der Destillationszone zugeordnet ist, vollständig innerhalb dieser Destillationszone liegt.

6. Verfahren nach einem der Ansprüche 1 bis 4, **dadurch gekennzeichnet, dass** es wenigstens eine Zone selektiver Hydrierung innerhalb und wenigstens eine Hydrierungszone außerhalb der Destillationszone umfasst.

7. Verfahren nach einem der Ansprüche 1 bis 6, **dadurch gekennzeichnet, dass** der Katalysator ein Katalysator mit Träger ist, der wenigstens ein Edelmetall der Gruppe VIII umfasst.

8. Verfahren nach Anspruch 7, **dadurch gekennzeichnet, dass** der Katalysator ein Palladiumkatalysator ist.

## Claims

1. A process for the treatment of a feed comprising hydrocarbons containing 3 to 10 carbon atoms per molecule, including acetylenic and diolefinic hydrocarbons, by passing it into a distillation zone including a stripping zone and a rectification zone, which produces a vapour and a liquid, said distillation zone being in communication with at least one selective hydrogenation reaction zone constituted by at least one catalytic bed, in which hydrogenation of at least a portion of the acetylenic and diolefinic hydrocarbons contained in said feed is carried out in the presence of a gas stream rich in hydrogen, said process being **characterized in that** the feed for the reaction zone(s) is drawn off at the level of a draw-off plate and represents at least the major portion of the liquid flowing in the rectification zone, the major portion of the effluent from the reaction zone being reintroduced into the rectification zone to ensure continuity of distillation, **in that** at least one selective hydrogenation reaction zone is internal to the distillation zone and **in that** the distillation vapour is separated from the liquid to be hydrogenated for any reaction zone internal or external to said distillation zone.

2. A process according to claim 1, **characterized in that** the feed from the reaction zone represents the major portion of the liquid flowing in an intermediate level of the rectification zone.

3. A process according to claims 1 or 2, **characterized in that** it is carried out so as to cause an effluent which is highly depleted in acetylenic and/or diolefinic hydrocarbons containing at most 5 carbon atoms to leave the head of the distillation column and an effluent which is also depleted in said compounds to leave the bottom of the distillation column.

4. A process according to any one of claims 1 to 3, **characterized in that** each selective hydrogenation zone comprises 1 to 4 catalytic beds.

5. A process according to any one of claims 1 to 4, **characterized in that** the selective hydrogenation zone associated with the distillation zone is completely internal to said distillation zone.

6. A process according to any one of claims 1 to 5, **characterized in that** it comprises at least one selective hydrogenation zone internal to and at least one selective hydrogenation zone external of said distillation zone.

7. A process according to any one of claims 1 to 6, **characterized in that** the catalyst is a supported catalyst comprising at least one noble metal from group VIII.

8. A process according to claim 7, **characterized in that** the catalyst is a palladium catalyst.
